# EUROPEAN PATENT APPLICATION

(11) **EP 1 685 833 A1**
(43) Date of publication of application: **02.08.2006**
(21) Application number: 04819004.5
(22) Date of filing: 22.11.2004
(51) Int. Cl.: A61K 31/198, A61K 45/00, A61K 38/26, A61K 38/28, A61P 13/04, A61P 13/06, A61P 9/12, A61P 25/00, A61P 39/06, A61P 43/00

(54) **REMEDY FOR DIABETES**

(30) Priority: 21.11.2003 JP 2003392718
(71) Applicant: Ajinomoto Co., Inc., Tokyo 104-8315 (JP)
(72) Inventor: NISHITANI, Shinobu, c/o AJINOMOTO CO., INC., Tokyo 1048315 (JP); TAKEHANA, Kenji, c/o AJINOMOTO CO., INC., Kawasaki-shi, Kanagawa 2108681 (JP)
(74) Representative: Nicholls, Kathryn Margaret
(86) International application number: PCT/JP2004/017336
(87) International publication number: WO 2005/049006

(57) **Abstract**

A pharmaceutical composition herein provided comprises at least one branched chain amino acid selected from the group consisting of isoleucine, leucine and valine, combined with at least one drug selected from the group consisting of anti-diabetic agents, agents for treating or preventing obesity and agents for treating or preventing diabetic complications. This pharmaceutical composition is useful as an agent for preventing and/or treating diabetes, diabetic complications, hyperinsulinemia, sugar dysbolism, or obesity.

## Description

### Technical Field

The present invention relates to a pharmaceutical composition and more particularly to a pharmaceutical composition comprising a specific branched chain amino acid combined with a specific drug, in particular, a therapeutic agent for preventing or treating diabetes.

### Background Art

The diabetes is a dysbolism attributable to the absolute or relative deficiency of insulin as the only hypoglycemic hormone and mainly characterized by the continuous maintenance of a high blood sugar level. The maintenance of such a high blood sugar level condition would not only exacerbate the dysbolism due to the deficiency of insulin, but also become the cause of microangiopathy of, for instance, kidneys, nerves and/or retina as well as the angiopathy of great blood vessels such as arteriosclerosis and it is thus quite difficult for a person suffering from such a condition to ensure their healthy life. Accordingly, the goal in the control of diabetes is to rectify this high blood sugar level condition and to thus prevent the occurrence of any chronic complication and to arrest the progress thereof.

Until now, there have widely been used various hypoglycemic agents such as insulin-containing pharmaceutical preparations, agents for accelerating the secretion of insulin, insulin resistance-improving agents and α-glycosidase inhibitor in the clinical methods for treating high blood sugar level conditions. These hypoglycemic agents have been recognized to be effective, but each of them has a variety of problems to be solved. For instance, the effectiveness of agents for accelerating the secretion of insulin or insulin resistance-improving agents would be reduced in a patient suffering from diabetes and whose pancreas is considerably deteriorated in its ability to secrete insulin.

It would be considered to be effective to use such a hypoglycemic agent in combination with a drug showing a mechanism of action different from that of the former in order to compensate or eliminate the foregoing inconvenience of the hypoglycemic agent. However, the use of the existing hypoglycemic agents in combination would show only a limited effectiveness in the alleviation or elimination of the high blood sugar level condition, while taking into consideration the diverse pathemas of the diabetes.

One of the principal actions taking part in the hypoglycemic mechanism of insulin is as follows: the insulin can enhance the ability of peripheral cells to transfer sugar moieties and can, in turn, allow the peripheral cells to take in sugar components present in the blood to thus reduce the blood sugar level.

The following Patent Document 1 which was filed by the same applicant as that of this patent application discloses a pharmaceutical agent for treating the abnormality of glucose tolerance, which comprises, as an effective component, at least one member selected from the group consisting of leucine, isoleucine, and valine, but this never discloses the simultaneous use thereof with other pharmaceutical agents and the pharmaceutical composition of the present invention.
Patent Document 1: Japanese Un-Examined Patent Publication 2003-171271.

### Disclosure of the Invention

It is an object of the present invention to provide, for instance, a pharmaceutical agent usable as an excellent prophylactic or therapeutic agent for diabetes and, in particular, a pharmaceutical composition showing an excellent hyperglycemic condition-improving effect, which has never been accomplished by the use of any conventional hypoglycemic agent.

The present inventors have made deeper and wider studies to solve the foregoing problems, have found that the use of at least one member selected from the group consisting of isoleucine, leucine and valine in combination with a specific drug can provide an improved anti-diabetic effect and, in particular, a hypoglycemic effect or a conspicuous therapeutic effect in the treatment of diabetes as compared with the effect observed when the drug is used alone or without combining with such an amino acid and have thus completed the present invention.

There are provided the following inventions:
[1] A pharmaceutical composition comprising a combination of at least one branched chain amino acid selected from the group consisting of isoleucine, leucine and valine, with at least one drug selected from the group consisting of anti-diabetic agents, agents for treating or preventing obesity and agents for treating or preventing diabetic complications.
[2] The pharmaceutical composition as set forth in the foregoing item 1, which is applied to the treatments of diseases caused by hyperglycemia and used for preventing and/or treating the diseases.
[3] The pharmaceutical composition as set forth in the foregoing item 1, which is applied to the treatments of diabetes, diabetic complications, hyperinsulinemia, the abnormality of glucose tolerance and obesity and used for preventing and/or treating the diseases.

### Brief Description of the Drawings

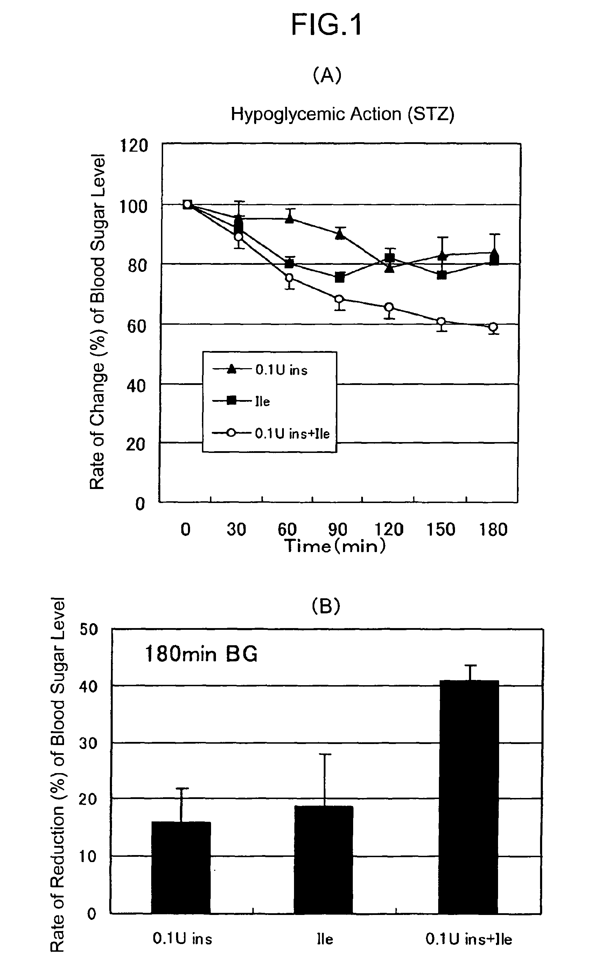

Fig. 1 is a graph showing the effect observed in Example 1, when simultaneously using isoleucine and an insulin-containing pharmaceutical preparation in the rats suffering from streptozotocin-induced type-1 diabetes as a model of type-1 diabetes. More specifically, (A) initially (time (t): 0), 0.1 U/kg of insulin (ins) was subcutaneously administered, and 1.5 g/kg of L-isoleucine (Ile) was orally administered or 0.1 U/kg of insulin and 1.5 g/kg of L-isoleucine (ins + Ile) were simultaneously administered, followed by the determination of the blood sugar level with the lapse of time to thus calculate the variation (%) of the blood sugar level relative to the initial blood sugar level (t: 0); and (B) the variation of the blood sugar level observed after 180 minutes was expressed in terms of the rate of reduction (%) in the blood sugar level relative to that (t = 0) initially observed (average ± standard deviation; N = 5 for each test group).

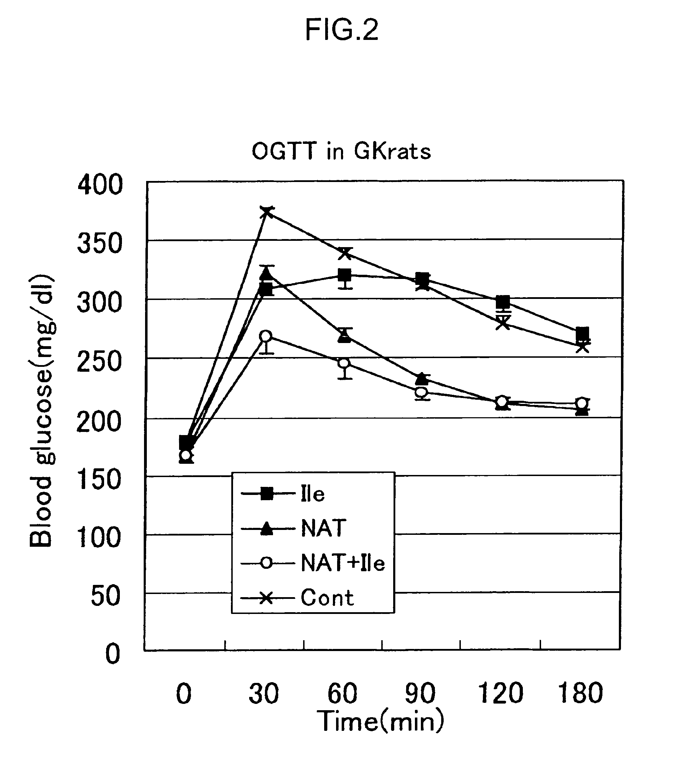

Fig. 2 is a graph showing the effect observed in Example 2, when simultaneously using isoleucine and Nateglinide in the GK rats as the type-II diabetes model. More specifically, initially (t: 0), 1 g/kg of glucose was orally administered, while simultaneously, 1.5 g/kg of L-isoleucine (Ile) was orally administered, or 50 mg/kg of Nateglinide (NAT) was orally administered, and 1.5 g/kg of L-isoleucine and 50 mg/kg of Nateglinide (NAT + Ile) were simultaneously administered, followed by the determination of the variation in the blood sugar level with the lapse of time (average±standard deviation; N = 5 for each test group).

### Best Mode for Carrying Out the Invention

The pharmaceutical composition according to the present invention comprises, in combination, at least one branched chain amino acid selected from the group consisting of isoleucine, leucine and valine, and at least one drug selected from the group consisting of anti-diabetic agents, agents for treating or preventing obesity and agents for treating or preventing diabetic complications (in other words, a combined drug) and the latter drug may be any one insofar as it can be combined with at least one branched chain amino acid selected from the group consisting of isoleucine, leucine and valine at the time of the administration thereof. Accordingly, the pharmaceutical composition for preventing and/or treating diabetes according to the present invention may be a single pharmaceutical preparation prepared by simultaneously forming, into a single pharmaceutical preparation, a mixture of at least one branched chain amino acid selected from the group consisting of isoleucine, leucine and valine and the foregoing drug, or may be a combination of at least two pharmaceutical preparations obtained by separately forming, into individual pharmaceutical preparations, at least one branched chain amino acid selected from the group consisting of isoleucine, leucine and valine, and the foregoing drug.

The foregoing at least one branched chain amino acid selected from the group consisting of isoleucine, leucine and valine is not necessarily in the free amino acid state, but may be in the form of, for instance, an inorganic acid salt, an organic acid salt or an ester derivative capable of being hydrolyzed in the living bodies. Alternatively, the branched chain amino acid selected from the foregoing group may be used in the form of a peptide obtained by combining, through peptide bonds, two or more of the branched chain amino acid selected from the group consisting of isoleucine, leucine and valine. At least one branched chain amino acid selected from the group consisting of isoleucine, leucine and valine usable herein may be L-isomers, D-isomers and DL-isomers, but preferably used herein are L-isomers since the naturally occurring amino acids are in their L-configuration.

Examples of anti-diabetic agents serving as drugs used in combination with at least one branched chain amino acid selected from the group consisting of isoleucine, leucine and valine in the present invention are insulin-containing pharmaceutical preparations, insulin derivatives, agents having insulinoid actions, insulin secretion-accelerating agents, insulin resistance-improving agents, biguanide-containing agents, glucogenesis-inhibitory agents, agents for inhibiting the absorption of sugar, agents for inhibiting the renal sugar re-absorption, β3 adrenalin-receptor agonists, glucagon-like peptide-1, glucagon-like peptide-1 analogues, glucagon-like peptide-1 receptor agonists, dipeptidyl peptidase IV inhibitors, glycogen-synthesizing enzyme kinase-3 inhibitory agents, glycogen phosphorylase-inhibitory agents or any combination thereof, examples of agents for treating or preventing obesity include antilipemics, anorexigenic agents, lipase-inhibitory agents or any combination thereof; and examples of agents for treating or preventing diabetic complications include hypotensive agents, peripheral circulation-improving agents, antioxidants, agents for treating diabetic neuropathy or any combination thereof. These agents may be used alone or in any combination of at least two of them.

Among them, preferably used herein are anti-diabetic agents, agents for treating or preventing obesity or any combination thereof, with the anti-diabetic agents being further preferred. More specifically, preferably used herein are those selected from the group consisting of β3 adrenalin-receptor agonists, glucagon-like peptide-1, glucagon-like peptide-1 analogues, glucagon-like peptide-1 receptor agonists, dipeptidyl peptidase IV inhibitors and anorexigenic agents, or preferably used herein are, for instance, those selected from the group consisting of insulin-containing pharmaceutical preparations, insulin secretion-accelerating agents, insulin resistance-improving agents, agents for inhibiting the absorption of sugar and agents for inhibiting the renal sugar re-absorption. Among them, particularly preferably used herein are insulin-containing pharmaceutical preparations and insulin secretion-accelerating agents.

When using a combination of at least one branched chain amino acid selected from the group consisting of isoleucine, leucine and valine with at least one of the foregoing drugs, the present invention embraces various embodiments of administration, for instance, these two kinds of components are simultaneously administered in the form of a single pharmaceutical preparation; they are simultaneously administered in the form of separate pharmaceutical preparations through the same or different administration routes; and they are separately administered, at a proper interval, in the form of separate pharmaceutical preparations through the same or different administration routes. Accordingly, the pharmaceutical agent of the present invention, which comprises a combination of at least one branched chain amino acid selected from the group consisting of isoleucine, leucine and valine with at least one of the foregoing drugs, may be administered as a single pharmaceutical preparation or in the form of a combination of different pharmaceutical preparations, as has already been discussed above.

Synergistic effects of preventing and/or treating the foregoing diseases would be accomplished when using at least one branched chain amino acid selected from the group consisting of isoleucine, leucine and valine and one or more of the foregoing drugs appropriately combined together. In addition, the combinatorial use of the foregoing two kinds of components would likewise permit the elimination or alleviation of the side effects of the foregoing drug simultaneously used as compared with those observed when the drug is used alone.

Specific compounds as the drugs used in combination with the foregoing amino acids and examples of diseases to be treated with the pharmaceutical composition of the invention will be illustrated below in detail, but the scope of the present invention is not restricted to these specific examples at all and each specific compound likewise includes its free form, and/or other pharmaceutically acceptable salts thereof.

Examples of insulin-containing pharmaceutical preparations are NPH, lente insulin, ultralente insulin and insulin preparations capable of being absorbed through the lung.

The term "insulin derivative(s)" used herein means a protein or a peptide derived from insulin, which possesses the action of insulin and examples thereof are Lispro, B10Asp and glargine.

The term "agent(s) having insulinoid actions" used herein means a substance other than the foregoing insulin derivative, which can show the physiological effects of insulin such as the effect of promoting the uptake of sugar into cells to some extent independent of insulin to thus show its hypoglycemic action and specific examples thereof include insulin receptor kinase-stimulating drugs (such as L-783281, TER-17411, CLX-0901 and KRX-613) and vanadium.

The term "insulin secretion-accelerating agent(s)" used herein means an agent which can act on the pancreatic β-cell to thus promote the secretion of insulin into the blood and to thus show the desired hypoglycemic action and specific examples thereof are sulfonyl urea-containing pharmaceutical preparations (such as Tolbutamide, Chloropropamide, Tolazamide, Acetohexamide, Gliclazide, Glimepiride, Glipizide, Glibenclamide (Glyburide)), Meglitinides (such as Nateglinide, Repaglinide and Michiglinide), and ATP-sensitive potassium channel-inhibitory agents other than the foregoing sulfonyl urea-containing pharmaceutical preparations and Meglitinides (such as BTS-67-582).

The term "insulin resistance-improving agent(s)" used herein means an agent which can enhance the effect of insulin in the target tissues of insulin to thus show its hypoglycemic action and specific examples thereof include peroxisome- proliferating agent-activation receptor (PPAR) γ-agonist (for instance, thiazolidine dione type compounds such as Pioglitazone, Rociglitazone, Troglitazone and Ciglitazone, or non-thiazolidine dione type compounds such as GI-262570, GW-1929 and JTT-501, YM-440), PPAR γ-antagonists (such as bisphenol A diglycidyl ether and LG-100641), PPAR α-agonists (for instance, Fibrate type compounds such as Clofibrate, Bezafibrate and Clinofibrate, or non-Fibrate type compounds), PPAR α/γ-agonists (such as KRP-297), retinoid X-receptor agonists (such as LG-100268), retinoid X-receptor antagonists (such as HX531) and protein tyrosine phosphatase-1B-inhibitory agents (such as PTP-112).

The term "biguanide-containing agent(s)" used herein means an agent capable of showing the hypoglycemic action through the glucogenesis-inhibitory action in the liver, the anaerobic glucolytic action-promoting effect or the insulin resistance-improving effect in the periphery and examples thereof are Metformin, Fenformin and Buformin.

The term "glucogenesis-inhibitory agent(s)" used herein means an agent which can show the hypoglycemic action mainly through the inhibition of glucogenesis and examples thereof include glucagon secretion-inhibitory agents (such as M&B 39890A), glucagon receptor antagonists (such as CP-99711, NNC-92-1687, L-168049 and BAY27-9955), and glucose-6-phosphatase-inhibitory agents.

The term "agent(s) for inhibiting the sugar absorption" used herein means an agent which can inhibit the digestion of carbohydrates contained in foods in the digestive tract with the help of an enzyme and, in turn, inhibits or delays the absorption of sugar within the body to thus show the hypoglycemic action and examples thereof are α-glucosidase-inhibitory agents (such as Acarbose, Bogribose, and Miglitol), and α-amylase-inhibitory agents (such as AZM-127).

The term "agent(s) for inhibiting the renal sugar re-absorption" used herein means an agent which can show the hypoglycemic action through the inhibition of any re-absorption of sugar in the renal tubules and examples thereof are sodium-dependent glucose transporter-inhibitory agents (such as T-1095 and Phlorhizin).

The term "β3 adrenalin-receptor agonist(s)" used herein means a substance which can show an effect of alleviating obesity and hyperinsulinemia by stimulating β3 adrenalin-receptor in lipids and promoting the oxidation of fatty acids to thus induce the consumption of energy and examples thereof include CL-316243 and TAK-677.

Examples of glucagon-like peptide-1 analogues are Excendin-4 and NN-2211; examples of glucagon-like peptide-1 receptor agonists include AZM-134; and examples of dipeptidyl peptidase IV inhibitors include NVP-DPP-728. These glucagon-like peptide-1 analogues, glucagon-like peptide-1 receptor agonists, dipeptidyl peptidase IV inhibitors and glucagon-like peptide-1 are substances each capable of showing the diabetes-alleviation effect through the imitation or enhancement of the action of glucagon-like peptide-1 in the cell.

The term "aldose reductase-inhibitory agent(s)" used herein means, among the agents favorably used in the treatment of diabetic complications, one which can reduce the amount of sorbitol excessively accumulated within cells by inhibiting the action of an aldose reductase. The accumulation of excess sorbitol in cells is observed for the tissue suffering from such diabetic complications and caused by the acceleration of the polyol-metabolic pathway due to the sustained high blood sugar level condition. Examples of such agents are Epalrestat, Tolrestat, Phydarestat and Zenerestat.

The term "agent(s) for inhibiting the formation of a terminal glycation product" used herein means, among the agents favorably used in the treatment of diabetic complications, one capable of alleviating the damages of cells by inhibiting the production of any terminal glycation product, which may be promoted by the sustained high blood sugar level condition in a patient suffering from diabetes. Examples thereof include NNC-39-0028 and OPB-9195.

Examples of glycogen-synthesizing enzyme kinase-3 inhibitory agents are SB-216763 and CHIR-98014; and examples of glycogen phosphorylase-inhibitory agents include CP-91149.

Examples of antilipemics are hydroxymethyl glutaryl coenzyme A reductase-inhibitory agents (such as Pravastatin, Simvastatin, Fluvastatin and Atorvastatin), Fibrate type drugs (such as Clofibrate, Bezafibrate and Simfibrate), and cholagogic agents.

Examples of anorexigenic agents are Sibutramine and Mazindol; and examples of lipase-inhibitory agents include Orlistat.

Examples of hypotensive agents are angiotensin converting enzyme-inhibitory agents (such as Captopril and Alacepril), angiotensin II receptor-antagonistic agents (such as Candesartan Sirexetil and Balsaltan), calcium antagonistic agents (such as Cilnidipin, Amlodipine and Nicardipine), diuretics (such as Trichlormethiazide and Spironolactone) and sympatholytic agents (such as Clonidine and Reserpine).

Examples of peripheral circulation-improving agents include ethyl eicosapentaenoate.

### Examples of antioxidants are lipoic acid and Probucol.

Examples of agents for treating diabetic neuropathy are Mecobalamin and Mexiletine hydrochloride.

Furthermore, hypoglycemic agents, antilipemics, agents for treating or preventing obesity, hypotensive agents, peripheral circulation-improving agents, antioxidants, agents for treating diabetic neuropathy or the like other than those specified above would fall within the scope of the present invention inasmuch as they are used in combination with at least one branched chain amino acid selected from the group consisting of isoleucine, leucine and valine.

Specific examples of diseases attributable to the high blood sugar level include diabetes, diabetic complications (such as retinopathy, neuropathy, nephrosis, ulcers, and diseases of great blood vessels), obesity, hyperinsulinemia, sugar dysbolism, hyperlipidemia, hypercholesterolemia, hypertriglyceridemia, lipid dysbolism, atherosclerosis, hypertension, congestive heart failure, edema, hyperuricuria, and gout.

For instance, when using the foregoing drugs in combination with at least one branched chain amino acid selected from the group consisting of isoleucine, leucine and valine, it is preferred to use at least one drug selected from the group consisting of insulin-containing pharmaceutical preparations, insulin derivatives, agents having insulinoid actions, insulin secretion-accelerating agents, insulin resistance-improving agents, biguanide-containing agents, glucogenesis-inhibitory agents, agents for inhibiting the absorption of sugar, agents for inhibiting the renal sugar re-absorption, β3 adrenalin-receptor agonists, glucagon-like peptide-1, glucagon-like peptide-1 analogues, glucagon-like peptide-1 receptor agonists, dipeptidyl peptidase IV inhibitors, glycogen-synthesizing enzyme kinase-3 inhibitory agents, glycogen phosphorylase-inhibitory agents, anorexigenic agents, or lipase-inhibitory agents in combination with the foregoing at least one branched chain amino acid, for the treatment of diabetes. More preferably, the foregoing at least one branched chain amino acid is used in combination with at least one drug selected from the group consisting of insulin-containing pharmaceutical preparations, insulin derivatives, agents having insulinoid actions, insulin secretion-accelerating agents, insulin resistance-improving agents, biguanide- containing agents, glucogenesis-inhibitory agents, agents for inhibiting the absorption of sugar, agents for inhibiting the renal sugar re-absorption, β3 adrenalin-receptor agonists, glucagon-like peptide-1, glucagon-like peptide-1 analogues, glucagon-like peptide-1 receptor agonists, dipeptidyl peptidase IV inhibitors, glycogen-synthesizing enzyme kinase-3 inhibitory agents and glycogen phosphorylase-inhibitory agents. Most preferably, the foregoing at least one branched chain amino acid is used in combination with at least one drug selected from the group consisting of insulin-containing pharmaceutical preparations, insulin derivatives, agents having insulinoid actions, insulin secretion-accelerating agents, insulin resistance-improving agents, biguanide-containing agents, glucogenesis-inhibitory agents, agents for inhibiting the absorption of sugar and agents for inhibiting the renal sugar re-absorption.

Similarly, when using the foregoing drugs in combination with at least one branched chain amino acid selected from the group consisting of isoleucine, leucine and valine, it is preferred to use at least one drug selected from the group consisting of insulin-containing pharmaceutical preparations, insulin derivatives, agents having insulinoid actions, insulin secretion-accelerating agents, insulin resistance-improving agents, biguanide- containing agents, glucogenesis-inhibitory agents, agents for inhibiting the absorption of sugar, agents for inhibiting the renal sugar re-absorption, β3 adrenalin-receptor agonists, glucagon-like peptide-1, glucagon-like peptide-1 analogues, glucagon-like peptide-1 receptor agonists, dipeptidyl peptidase IV inhibitors, aldose reductase-inhibitory agents, agents for inhibiting the formation of a terminal glycation product, glycogen-synthesizing enzyme kinase-3 inhibitory agents, glycogen phosphorylase-inhibitory agents, antilipemics, anorexigenic agents, lipase-inhibitory agents, hypotensive agents, peripheral circulation-improving agents, antioxidants and agents for treating diabetic neuropathy in combination with the foregoing at least one branched chain amino acid, in the treatment of diabetic complications. More preferably, the foregoing at least one branched chain amino acid is used in combination with at least one drug selected from the group consisting of aldose reductase-inhibitory agents, agents for inhibiting the formation of a terminal glycation product, hypotensive agents; peripheral circulation-improving agents, antioxidants and agents for treating diabetic neuropathy.

Furthermore, in the treatment of obesity, it is preferred to use the foregoing at least one branched chain amino acid in combination with at least one drug selected from the group consisting of insulin-containing pharmaceutical preparations, insulin derivatives, agents having insulinoid actions, insulin secretion-accelerating agents, insulin resistance-improving agents, biguanide-containing agents, glucogenesis-inhibitory agents, agents for inhibiting the absorption of sugar, agents for inhibiting the renal sugar re-absorption, β3 adrenalin-receptor agonists, glucagon-like peptide-1, glucagon-like peptide-1 analogues, glucagon-like peptide-1 receptor agonists, dipeptidyl peptidase IV inhibitors, glycogen-synthesizing enzyme kinase-3 inhibitory agents, glycogen phosphorylase-inhibitory agents, anorexigenic agents and lipase-inhibitory agents. More preferably, the foregoing at least one branched chain amino acid is used in combination with at least one drug selected from the group consisting of β3 adrenalin-receptor agonists, anorexigenic agents and lipase-inhibitory agents.

Regarding the pharmaceutical agents comprising at least one branched chain amino acid selected from the group consisting of isoleucine, leucine and valine; and the foregoing drug in combination, according to the present invention, these two kinds of components may, for instance, be combined together, by preparing a single pharmaceutical preparation comprising them in combination; or separately forming into individual preparations and then packaging these preparations in the form of a kit, or separately packaging these preparations. The mixing ratio of at least one branched chain amino acid selected from the group consisting of isoleucine, leucine and valine to the foregoing drug may widely vary depending on various factors such as the desired doses of these components and the kinds of pharmaceutically acceptable carriers used, but the amount of the foregoing drug preferably ranges from about 0.000001 to 1 per unit mass of the at least one branched chain amino acid selected from the group consisting of isoleucine, leucine and valine, in the both cases wherein these two kinds of components are formed into a single preparation and they are separately formed into individual preparations.

When applying the pharmaceutical preparation to a patient as a combined single drug, it may be administered in such a manner that the dose of each component falls within the range specified above. Alternatively, when these two kinds of effective components are administered as separate pharmaceutical preparations, the average ratio of these effective components may be adjusted such that the ratio falls within the range specified above.

More specifically, the pharmaceutical preparation of the present invention may comprise about 0.01 to 10 g of the foregoing at least one branched chain amino acid selected from the group consisting of isoleucine, leucine and valine and about 0.001 to 2000 mg of the foregoing drug per unitary preparation, provided that the amount of the latter may fall within the range of from 0.01 to 500U, in case of an insulin-containing pharmaceutical preparation, an insulin derivative, a glucagon- like peptide-1 or a glucagon-like peptide-1 analogue.

When using the pharmaceutical composition of the present invention, it may be administered through the oral, intravenous, subcutaneous or intramuscular route. The dose thereof may widely vary depending on various factors such as the symptom and age of each particular patient and the manner or route of administration of the composition, the foregoing at least one branched chain amino acid selected from the group consisting of isoleucine, leucine and valine is administered at a dose ranging from 0.1 to 30 g/kg/day, while the foregoing drug is administered at a dose ranging from 0.01 to 20000 mg/kg/day (provided that the dose ranges from 0.01 to 1000 U/kg/day in case of an insulin-containing pharmaceutical preparation, an insulin derivative, a glucagon-like peptide-1 or a glucagon-like peptide-1 analogue).

In this respect, when calculating the dose (intake) of the foregoing at least one branched chain amino acid selected from the group consisting of isoleucine, leucine and valine, the amount thereof is set at the level specified above in case where it is incorporated, as one of the effective components, into the drug used for treating or preventing the diseases and abnormalities to be handled according to the present invention and therefore, it is not necessary to take into consideration the amount of the foregoing branched chain amino acid ingested by or administered to a patient for the purposes other than the foregoing, for instance, as a result of his usual diet or eating habits or for the purpose of the treatment of other diseases. For instance, it is not necessary to subtract the amount (per day) of at least one branched chain amino acid selected from the group consisting of isoleucine, leucine and valine ingested through the usual diet from the foregoing dose of the effective component per day, specified above.

The pharmaceutical composition of the present invention can be formed into each desired pharmaceutical preparation according to the usual method. Examples of the dosage forms of the composition include injections, tablets, granules, fine granules, powders, capsules, creams and suppositories. In the preparation of these pharmaceutical preparations, they may comprise various carriers for the preparation thereof and examples thereof include lactose, glucose, D-mannitol, starch, crystalline cellulose, calcium carbonate, kaolin, hydroxypropyl cellulose, hydroxypropyl methyl cellulose, polyvinyl pyrrolidone, ethanol, carboxymethyl cellulose, calcium salt of carboxymethyl cellulose, magnesium stearate, talc, acetyl cellulose, sucrose, titanium oxide, benzoic acid, 4-hydroxy benzoic acid ester, sodium dehydroacetate, gum Arabic, tragacanth, methyl cellulose, yolk, surfactants, simple syrup, citric acid, distilled water, glycerin, propylene glycol, macrogol, sodium mono-hydrogen phosphate, sodium di-hydrogen phosphate, sodium phosphate, sodium chloride, phenol, thimerosal, and sodium hydrogen sulfite, which may appropriately be selected and used depending on the shapes of the foregoing pharmaceutical preparations.

The pharmaceutical composition of the present invention which comprises at least one branched chain amino acid selected from the group consisting of isoleucine, leucine and valine; and at least one drug other than the foregoing in combination or in the form of a mixture would ensure the achievement of an anti-diabetic effect which can never be achieved by the use of any conventional hypoglycemic agent and accordingly, the pharmaceutical composition of the present invention would be quite useful in the prevention or treatment of a variety of diseases related to or caused by the hyperglycemia.

The present invention will hereunder be described in more detail with reference to the following Examples, but the following Examples are herein given only for the illustration of the present invention. Thus, the present invention is not restricted to these specific examples at all.

### Example 1: Investigation of Effects Achieved by Simultaneous Use of Isoleucine (Ile) and Insulin-Containing Pharmaceutical Preparation (Ins) While Making Use of Rats Suffering from Streptozotocin-Induced Type-1 Diabetes

Streptozotocin (available from Wako Pure Chemical Co., Ltd., Osaka, Japan) dissolved in a citric acid buffer (pH 4.2) was administered, through the tail vein, to Sprague-Dawley male rats of 7-week-old in a rate of 75 mg/kg (body weight) and after one week, these animals were screened so as to select those having a blood sugar level of not less than 350 mg/dl and the animals thus screened were used as the models of rats suffering from streptozotocin-induced type-1 diabetes. To the streptozotocin-induced type-1 diabetic rats which had been fasted for 17 hours, there was orally administered 1.5 g/kg of L-isoleucine (a 6% solution; control: physiological saline) and immediately thereafter, 0.1 U/kg of insulin was subcutaneously administered to each animal. Subsequently, the blood sugar level of each animal was monitored with the lapse of time. The results thus obtained are plotted on Fig. 1.

The data shown in Fig. 1 clearly indicate that the test animal group containing isoleucine and insulin (0.1 U/kg) simultaneously administered thereto in advance shows conspicuous reduction of the blood sugar levels as compared with those observed for the animal group containing only insulin (0.1 U/kg) administered thereto in advance.

### Example 2: Investigation of Effects Achieved by Simultaneous Use of Leucine and Insulin Secretion-Promoting Agent (Nateglinide) While Making Use of GK Rats as Type-II Diabetic Model

GK Rats were purchased from Nippon CREA and those used herein were 21- to 25-week old. To GK rats which had been fasted for 17 hours, there were orally administered 50 mg/kg of Nateglinide and 1.5 g/kg of L-isoleucine separately or in combination and at the same time, 1 g/kg each of a glucose solution was orally administered. Then the blood sugar level and the insulin level in the plasma were monitored with the lapse of time. The results thus obtained are plotted on Fig. 2.

As will be seen from the data plotted on Fig. 2, the test animal group containing L-isoleucine (1.5 g/kg) and Nateglinide (50 mg/kg) simultaneously administered thereto in advance shows conspicuous reduction of the blood sugar levels as compared with those observed for the animal group containing only Nateglinide (50 mg/kg) administered thereto in advance and the animal group containing only the compound 1 (1.5 g/kg) administered thereto in advance.

As will be clear from the foregoing result, the pharmaceutical composition of the present invention would be quite useful in the treatment of a variety of diseases attributable to hyperglycemia. More specifically, the use of at least one branched chain amino acid selected from the group consisting of isoleucine, leucine and valine appropriately combined with the foregoing drug would permit the achievement of a significantly high anti-diabetic effect as compared with those observed when separately using the foregoing at least one branched chain amino acid selected from the group consisting of isoleucine, leucine and valine, and the at least one drug other than the foregoing. Consequently, the pharmaceutical composition of the present invention is quite useful as an agent for preventing and/or treating diabetes, diabetic complications, hyperinsulinemia, sugar dysbolism or obesity.

## Claims

1. A pharmaceutical composition comprising at least one branched chain amino acid selected from the group consisting of isoleucine, leucine and valine, combined with at least one drug selected from the group consisting of anti-diabetic agents, agents for treating or preventing obesity and agents for treating or preventing diabetic complications.

2. The pharmaceutical composition as set forth in claim 1 wherein the anti-diabetic agent is selected from the group consisting of insulin-containing pharmaceutical preparations, insulin derivatives, agents having insulinoid actions, insulin secretion-accelerating agents, insulin resistance-improving agents, biguanide-containing agents, glucogenesis-inhibitory agents, agents for inhibiting the absorption of sugar, agents for inhibiting the renal sugar re-absorption, β3 adrenalin-receptor agonists, glucagon-like peptide-1, glucagon-like peptide-1 analogues, glucagon-like peptide-1 receptor agonists, dipeptidyl peptidase IV inhibitors, glycogen-synthesizing enzyme kinase-3 inhibitory agents and glycogen phosphorylase-inhibitory agents; the agent for treating or preventing obesity is selected from the group consisting of antilipemics, anorexigenic agents and lipase-inhibitory agents; and the agent for treating or preventing diabetic complications is selected from the group consisting of hypotensive agents, peripheral circulation-improving agents, antioxidants and agents for treating diabetic neuropathy.

3. The pharmaceutical composition as set forth in claim 1 or 2, which is applied to the treatments of diseases caused by the hyperglycemia and used for preventing and/or treating the diseases.

4. The pharmaceutical composition as set forth in claim 1 or 2, which is applied to the treatments of diabetes, diabetic complications, hyperinsulinemia, the abnormality of glucose tolerance or obesity and used for preventing and/or treating the diseases.

5. The pharmaceutical composition as set forth in any one of claims 1 to 4, wherein the drug is selected from the group consisting of anti-diabetic agents, agents for treating or preventing obesity and combination thereof.

6. The pharmaceutical composition as set forth in any one of claims 1 to 4, wherein the drug is an anti-diabetic agent.

7. The pharmaceutical composition as set forth in any one of claims 1 to 4, wherein the drug is selected from the group consisting of β3 adrenalin-receptor agonists, glucagon-like peptide-1, glucagon-like peptide-1 analogues, glucagon-like peptide-1 receptor agonists, dipeptidyl peptidase IV inhibitors and anorexigenic agents.

8. The pharmaceutical composition as set forth in any one of claims 1 to 4, wherein the drug is selected from the group consisting of insulin-containing pharmaceutical preparations, insulin secretion-accelerating agents, insulin resistance-improving agents, agents for inhibiting the absorption of sugar and agents for inhibiting the renal sugar re-absorption.

9. The pharmaceutical composition as set forth in any one of claims 1 to 4, wherein the drug is an insulin-containing pharmaceutical preparation or an insulin secretion-accelerating agent.

10. The pharmaceutical composition as set forth in claim 9, wherein the drug is an insulin-containing pharmaceutical preparation.

11. The pharmaceutical composition as set forth in claim 9, wherein the drug is an insulin secretion-accelerating agent.

12. The pharmaceutical composition as set forth in any one of claims 1 to 11, wherein the branched chain amino acid is isoleucine.

13. The pharmaceutical composition as set forth in any one of claims 1 to 12, which is in the dosage form comprising 0.1 to 30 g/kg/day of the branched chain amino acid and 0.01 to 20000 mg/kg/day of the drug.

14. The pharmaceutical composition as set forth in claim 13, wherein the branched chain amino acid and the drug are in the form of a single pharmaceutical preparation.

15. The pharmaceutical composition as set forth in claim 13, wherein the branched chain amino acid and the drug are in the form of separate pharmaceutical preparations.
